(19) Europäisches Patentamt
European Patent Office
Office européen des brevets

(11) Numéro de publication: **0 203 874 B1**

(12) **FASCICULE DE BREVET EUROPEEN**

(45) Date de publication du fascicule du brevet: **01.08.90**

(51) Int. Cl.⁵: **C07C 263/00**, C07D 251/34, C08G 18/00

(21) Numéro de dépôt: **86420123.1**

(22) Date de dépôt: **15.05.86**

(54) **Procédé de stabilisation de solutions organiques de polyisocyanates.**

(30) Priorité: **21.05.85 FR 8507817**

(43) Date de publication de la demande:
**03.12.86 Bulletin 86/49**

(45) Mention de la délivrance du brevet:
**01.08.90 Bulletin 90/31**

(84) Etats contractants désignés:
**AT BE CH DE GB IT LI LU NL SE**

(56) Documents cités:
**BE-A- 676 738**

**CHEMICAL ABSTRACTS, vol. 98, 1983, page 43, abstract no. 108 378s, Columbus, Ohio, US**

**Le dossier contient des informations techniques présentées postérieurement au dépôt de la demande et ne figurant pas dans le présent fascicule.**

(73) Titulaire: **RHONE-POULENC CHIMIE, 25, quai Paul Doumer, F-92408 Courbevoie Cédex(FR)**

(72) Inventeur: **Robin, Jean, 21, rue Duguesclin, F-69006 Lyon(FR)**

(74) Mandataire: **Vignally, Noel et al, RHONE-POULENC INTERSERVICES Service Brevets Chimie Centre de Recherches de Saint-Fons B.P. 62, F-69192 Saint-Fons Cedex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention est relative à un procédé de stabilisation de solutions de polyisocyanates dans les solvants organiques vis-à-vis de la formation de précipités au cours du stockage.

En raison de leurs propriétés chimiques et mécaniques les revêtements à base de polyuréthannes connaissent un développement considérable comme peintures, laques, vernis pour des supports variés tels que les métaux, le bois, le béton, les polymères. L'obtention de ces revêtements polyuréthanniques est le plus souvent réalisée par réaction sur le support choisi, d'un composé polyhydroxylé (polyols, poléthers polyhydroxylés, polyesters polyhydroxylés par exemple) avec un composé comportant une pluralité de groupes isocyanates en présence d'adjuvants destinés à faciliter la réaction et le durcissement du film obtenu. La réactivité des composés à hydroxyles libres et des composés à groupes ioscyanates libres implique que la réunion des deux protagonistes de la réaction ne soit réalisée que peu de temps avant l'application, par exemple dans la chambre de mélangeage d'un appareil de pulvérisation dans laquelle ils sont alimentés séparément.

L'obtention du revêtement polyuréthannique passe donc, dans ces conditions, par la mise en oeuvre de deux composants séparés qui justifie les noms de compositions de revêtements (vernis, peintures, laques) à deux constituants ou bi-composants donnés à ces compostions. D'une manière générale l'un des deux composants comprend le composé polyhydroxylé auquel peuvent être ajoutés des adjuvants divers tels que des pigments, des agents épaississants, des agents fluidifiants, des stabilisants (agents anti-UV par exemple), des solvants, et éventuellement un catalyseur de polyaddition et l'autre composant est constitué par une solution d'un ou plusieurs composés à groupes isocyanates libres dans un solvant organique ou un mélange de solvants organiques destiné(s) à faciliter la mise en oeuvre du composant polyisocyanate et son mélangeage avec le composant polyhydroxylé. Les solvants utilisés à cet effet sont des composés liquides dans les conditions normales de pression et de température et qui doivent être dépourvus de groupes fonctionnels susceptibles de réagir avec les groupes isocyanates et notamment de groupes fonctionnels à hydrogène actif (suivant Zerewitinoff) tels que les groupes amino, hydroxyle, mercaptan, carboxyle. Il importe aussi pour la stabilité de ces solutions que les solvants utilisés soient exempts d'eau, condition qu'il est particulièrement difficile de respecter au plan industriel sans recourir à des moyens onéreux pour éliminer les dernières traces d'eau. Par ailleurs, quels que soient les soins apportés à l'élimination de l'eau il est difficile de conserver aux solvants leur caractère anhydre. En effet de l'eau peut être apportée accidentellement, en particulier à partir de la vapeur d'eau atmosphérique dans le cas de solvants hydrophiles. Or il a été constaté que la présence de traces d'eau dans les solvants organiques de polyisocyanates provoquent au cours de leur stockage la formation de précipités qui nuisent à leur présentation, à leur mise en œuvre et peuvent porter préjudice à l'aspect du revêtement final.

Le brevet BE-A 676 738 décrit une composition d'uréthanne vulcanisable ayant une plus grande durée de stockage admissible, comprenant un prépolymère d'uréthanne à fonctions isocyanates libres, un agent d'allongement de chaîne constitué par une aryldiamine et un glycol ou un aminoalcanol, et un composé organique d'étain essentiellement choisi parmi les carboxylates de dialkylétain IV ou de monoalkylétain IV ou un carboxylate d'étain II.

Le résumé des Chemicals Abstracts 98, 108 378s (1983) décrit des compositions de prépolymère obtenu à partir d'isophorone diisocyanate et d'un polyol, ayant une bonne stabilité au stockage due à l'addition de composés de dioctylétain.

Ainsi l'industrie est à la recherche d'un moyen permettant d'éviter d'une part la mise en œuvre de procédés coûteux d'élimination des traces d'eau des solvants utilisés pour l'obtention des solutions organiques de polyisocyanates et d'autre part la formation de précipités dans ces solutions contenant des traces d'eau. La présente invention a précisément pour objet un procédé de stabilisation de solutions organiques de polyisocyanates vis-à-vis de la formation de précipités et les compositions ainsi obtenues.

Plus spécifiquement la présente invention a pour objet un procédé de stabilisation de solutions de polyisocyanates dans des solvants organiques inertes, vis-à-vis de la formation de précipités caractérisé en ce qu'on leur ajoute une quantité efficace d'au moins un dérivé organostannique soluble dans le solvant organique.

On a constaté, de façon inattendue, que la présence d'un dérivé organostannique soluble dans la solution organique de polyisocyanate inhibe la formation de précipités au cours de stockage.

Comme dérivés de l'étain on utilise de préférence des composés organostanniques solubles dans les solvants utilisés habituellement pour mettre en solution le composant polyisocyanate des compositions de revêtements polyuréthanniques qui répondent à la formule générale:
dans laquelle:

- R représente un radical hydrocarboné ayant de 1 à 30 atomes de carbone, éventuellement substitué par un atom d'halogène ou un groupe fonctionnel inerte vis-à-vis des groupes isocyanates, les différents radicaux R pouvant être identiques ou différents;
- X représente un atome d'halogène, un reste acyloxy, un reste sulfonyloxy, un reste d'un acide minéral oxygéné, un reste de formule $-Z-Sn(R)_3$ dans laquelle Z représente un atome d'oxygène ou de soufre et R a la signification donnée ci-avant; un reste de formule $R'-Z$ dans laquelle R' représente un radical alkyle comportant 1 à 30 atomes de carbone, des radicaux cycloalkyles comportant de 5 à 12 atomes de carbone, des radicaux arylalkyles comportant 1 à 10 atomes de carbone dans le reste alkyle, éventuellement substitués par un ou plusieurs

atomes d'halogène et/ou groupes fonctionnels inertes vis-à-vis des groupes isocyanates, tels que alkoxy, carbonyloxyalkyle, nitro ou acyle.

Plus spécifiquement dans la formule (I):

– R représente un radical alkyle comportant de 1 à 30 atomes de carbone et de préférence de 1 à 20 atomes de carbone tel que les radicaux méthyle, éthyle, propyle, isopropyle, n-butyle, isobutyle, tertiobutyle, n-pentyle, isoamyle, n-hexyle, n-heptyle, éthyl-2 hexyle, n-octyle, décyles, dodécyles, octadécyles; des radicaux cycloalkyles comportant de 5 à 15 atomes de carbone et de préférence de 5 à 12 atomes de carbone tels que les radicaux cyclopentyle, cyclohexyle, méthyl-2 cyclohexyle, méthyl-4 cyclohexyle, diméthyl-2,3 cyclohexyle, cyclododécyle; des radicaux aryles tels que phényle, naphtyle, toluyle, xylyle; des radicaux arylalkyles comportant de 1 à 10 atomes de carbone dans le reste alkyle tels que les radicaux benzyle, alpha-phényléthyle, β-phényléthyle. Ces radicaux hydrocarbonés peuvent être substitués par un ou plusieurs atomes d'halogènes tels que chlore, brome ou fluor et/ou par des groupes fonctionnels inertes tels que: alkoxy (méthoxy, éthoxy, propoxy, butoxy); carbonyloxyalkyle (carbonyloxyméthyle, carbonyloxyéthyle); nitro; acyle (acétyle, propionyle, benzoyle).

– X représente un atome de chlore, de brome, de fluor; un reste acyloxy de formule $R_1$–COO dans laquelle $R_1$ représente un radical alkyle, cycloalkyle, aryle, arylalkyle tels que ceux définis pour R; un reste acyloxy de formule générale:

$$(R_2OOC)_p–R_3–COO–$$

dans laquelle:

. $R_2$ représente un radical alkyle inférieur ou un reste $(R)_3Sn$–, R ayant la signification donnée ci-avant;

. p est 1 à 2

. $R_3$ est un reste hydrocarboné aliphatique saturé, cycloaliphatique saturé ou aromatique di- ou trivalent tel que les restes tétraméthylène, pentaméthylène, hexaméthylène, dodécaméthylène, o-phénylène, métaphénylène, paraphénylène.

– X représente encore un reste sulfonyloxy $R_1$–$SO_3$– ($R_1$ ayant la signification donnée ci-avant); un reste de formule:

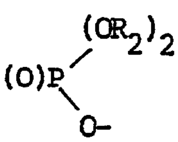

dans laquelle $R_2$ a la signification donnée ci-avant, les deux radicaux $R_2$ pouvant être identiques ou différents; un reste de formule:

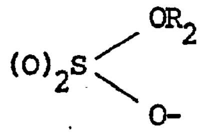

dans laquelle $R_2$ a la signification donnée ci-avant.

Les composés organostanniques qui conviennent à la mise en œuvre du procédé selon la présente invention peuvent être choisis parmi ceux cités dans l'article de R. K. INGHAM et al, Chem. Rev. 60 pages 459 à 539 (1960). On peut citer à titre d'exemples non limitatifs: le chlorure de tribenzylétain, le fluorure de tribenzylétain, le chlorure de tributylétain, le bromure de tricyclohexylétain, le chlorure de triisubutylétain, le bromure de tricotylétain, le chlorure de triphénylétain, le chlorure de tris(paracarbométhoxyphényl)étain, le chlorure de tris-(p-chlorophényl)étain, le chlorure de bis(carbométhoxy-2 éthyl)propylétain, le bromure de butyldicyclohexylétain, le bromure de décydiméthylétain, le chlorure de dibutylphénylétain, le bromure de diméthyloctylétain, le bromure de phényldioctylétain, le chlorure de diphényloctylétain, le chlorure de dodécyldiéthylétain, le méthoxytributylétain, le méthoxytrioctylétain, le butoxytributylétain, le (n-octylthio)triéthylétain, le (t-butylthio)triéthylétain, le laurate de dibutyldodécylétain, le phtalate de bis-(tributylétain), l'acétate de dodécyldiéthylétain, le sulfate de bis(triéthylétain), le laurate de dibutyl-(dodécylthio)étain, le phtalate de bis-(dibutylméthoxyétain), l'oxyde de triéthylétain, l'oxyde de tributylétain, le sulfure de bis(tributylétain), le sulfure de bis(triphénylétain).

La quantité de dérivés organostanniques exprimée en pourcentage du poids de polyisocyanate peut varier dans de larges limites en fonction de la nature du polyisocyanate, de celle du solvant et de celle du dérivé organostannique. La quantité minimale nécessaire à l'inhibition de la formation des précipités peut être déterminée au moyen de tests simples. Il n'y a pas de quantité supérieure critique mais en général il est inutile d'utiliser une quantité de dérivé organostannique représentant plus de 5% du poids du polyisocyanate. Des quantités représentant de $1 \times 10^{-4}$ à 2% et de préférence de $1 \times 10^{-3}$ à 1% du poids du polyisocyanate conviennent bien.

Les solutions de polyisocyanates qui peuvent être stabilisées par le procédé de l'invention sont celles utilisées habituellement dans les compositions de revêtements bi-composants. Les solvants sont ceux auxquels on a recours usuellement dans ce type de compositions. On trouvera des exemples de ces solvants cités dans les ouvrages généraux concernant les revêtements polyuréthanniques tels que ceux de J. H. SAUNDERS et al "POLYURETHANES – CHEMISTRY AND TECHNOLOGY I. Chemistry" Interscience Publishers (1963) et "II. Technology" Interscience Publishers (1964) et de K. WEIGEL "POLYURETHAN LACKE" publié par Holzverlag (1966). Les solvants suivants peuvent être mis en œuvre: des esters tels que

l'acétate d'éthyle, l'acétate de butyle, l'acétate d'amyle, l'acétate de l'éther monométhylique de l'éthylèneglcol, l'acétate d'éthylèneglycol, l'acétate de méthoxybutyle ; des cétones telles que la cyclohexanone, la méthylisobutylcétone, la méthyléthylcétone ; des hydrocarbures halogénés tels que le chlorure de méthylène, le trichloroéthylène ; des hydrocarbures aromatiques tels que le benzène, le toluène, les xylènes et leurs dérivés halogénés; des étheroxydes tels que le méthylisopropyléther, le méthylisobutyléther, les éthers diméthyliques ou diéthyliques des mono- et diéthylèneglycols. Le procédé selon l'invention s'applique naturellement à des solutions de polyisocyanates dans des mélanges de deux ou plus de deux des solvants précités. Il s'applique tout particulièrement bien aux solutions comportant des solvants hydrophiles.

Les polyisocyanates présents dans les solutions organiques sont ceux utilisés ou utilisables dans les compositions de revêtements. Des exemples de tels polyisocyanates sont donnés dans les ouvrages de SAUNDERS et al et de WEIGEL cités plus haut. Plus spécifiquement et non limitativement on peut faire appel à des diisocyanates aromatiques tels que le toluènediisocyanate (TDI), le bis(isocyanatophényl)méthane, le bis(méthyl-3 isocyanato-4 phényl)méthane, les xylylènediisocyanates, le naphtalènediisocyanate-1,5 ; des diisocyanates aliphatiques ou cycloaliphatiques tels que le pentaméthylènediisocyanate, l'hexaméthylènediisocyanate, l'octaméthylènediisocyanate, le diisocyanato-1,4 cyclohexane, le bis(isocyanato-4 cyclohexyl)méthane, l'isophoronediisocyanate ; des polyisocyanates à structure biuret obtenus par réaction d'un diisocyanate notamment aliphatique ou aliphatique avec l'eau ou un composé à hydrogène actif ; on peut citer à titre d'exemples non limitatifs les polyisocyanates à structure biuret dérivés de l'hexaméthylènediisocyanate et du diisocyanato-1,4 cyclohexane ; des polyisocyanates à structure isocyanurate obtenus par cyclotrimérisation catalytique des polyisocyanates aliphatiques, cycloaliphatiques ou aromatiques; on peut en particulier faire appel à des polyisocyanatopolyisocyanurates obtenus par cyclotrimétrisation de di- ou polyisocyanates tels que ceux cités dans le brevet européen 0057.653 ; peuvent encore être utilisés les "adducts" à groupes isocyanates libres obtenus par condensation d'un excès d'un di- ou polyisocyanate avec un ou plusieurs composés à hydrogènes actifs tels qu'un polyol: glycols (éthylèneglycol, propylèneglycol); polyols (triméthylolpropane); étherdiol (diéthylèneglycol; dipropylèneglycol), à caractère non polymérique. On entend par là des composés dont le poids moléulaire est insuffisant pour leur conférer un caractère filmogène.

D'un point de vue pratique la stabilisation des compositions organiques de polyisocyanates est effectuée simplement par addition du dérivé organostannique soit au solvant avant dissolution du polyisocyanate, soit à la solution de polyisocyanate. On ne sortirait pas du cadre de l'invention en ajoutant une quantité efficace de composé organostannique au polyisocyanate avant mise en solution.

Les exemples suivants illustrent l'invention et montrent comment elle peut être mise en pratique.

EXEMPLES 1 à 10

Dans des flacons de 100 ml on charge :

- 12 g d'un trimère isocyanurate de l'hexaméthylènediisocyanate exempt de ce dernier
- 14 g d'acétate d'éthyle,
- 14 g de xylène,
- 500 parties en poids par million par rapport au polyisocyanate des dérivés organostanniques cités ci-après.

Les flacons sont bouchés et conservés à 60°C en même temps qu'un flacon témoin ne contenant pas de dérivé de l'étain. Les flacons sont conservés dans ces conditions pendant trois mois et examinés tous les dix jours. On constate qu'au dixième jour la solution exempte de dérivé organostannique est trouble alors qu'après trois mois le contenu des autres flacons est resté limpide. Les composés organostanniques suivants ont été testés.

exemple 1 : oxyde de tributylétain
exemple 2 : chlorure de tributylétain
exemple 3 : benzoate de tributylétain
exemple 4 : phosphate de diéthyle et de tributylétain
exemple 5 : phosphate de tris(tributylétain)
exemple 6 : acétate de lauryldiéthylétain
exemple 7 : laurate de triéthylétain
exemple 8 : méthoxytributylétain
exemple 9 : acétate de méthoxydibutylétain
exemple 10: triéthyl(isobutythio)étain.

## Revendications

1. Procédé de stabilisation de polyisocyanates dans des solvants organiques inertes, vis-à-vis de la formation de précipités, caractérisé en ce qu'on leur ajoute une quantité efficace d'au moins un dérivé organostannique soluble dans le solvant organique et qui répond à la formule générale:

$$(R)_3 - Sn - X \quad (I)$$

dans laquelle:

– R représente un radical hydrocarboné ayant de 1 à 30 atomes de carbone, éventuellement substitué par un atome d'halogène ou un groupe fonctionnel inertes vis-à-vis des groupes isocyanates tel que alkoxy, carbonyloxyalkyle, nitro, ou acyle, les différents radicaux pouvant être identiques ou différents;

– X représente un atome d'halogène; un reste acyloxy; un reste sulfonyloxy; un reste d'acide minéral oxygéné; un reste de formule $-Z-Sn(R)_3$ dans laquelle Z représente un atome d'oxygène ou de soufre et R a la signification donnée ci-avant; un reste de formule $R'-Z$ dans laquelle R' représente un radical alkyle comportant 1 à 30 atomes de carbone, des radicaux cycloalkyles comportant de 5 à 12 atomes de carbone, des radicaux arylalkyles comportant 1 à 10 atomes de carbone dans le reste alkyle, éventuellement substitués par un ou plusieurs atomes d'halogène et/ou groupes fonctionnels inertes vis-à-vis des groupes isocyanates, tels que alkoxy, carbonyloxyalkyle, nitro ou acyle.

2. Procédé selon la revendication 1, caractérisé en ce que l'on fait appel à des composés organostanniques de formule (I) dans laquelle:

– R représente un radical alkyle comportant de 1 à 30 atomes de carbone; des radicaux cycloalkyles comportant de 5 à 12 atomes de carbone; des radicaux aryles; des radicaux arylalkyles comportant de 1 à 10 atomes de carbone dans le reste alkyle, éventuellement substitués par un ou plusieurs atomes d'halogène et/ou groupes fonctionnels inertes vis-à-vis des groupes isocyanates tels que alkoxy, carbonyloxyalkyle, nitro ou acyle;

– X représente:

– un atome de fluor, de chlore ou de brome;

– un reste acyloxy de formule $R_1$–COO dans laquelle $R_1$ représente un radical alkyle, cycloalkyle, aryle, arylalkyle tels que ceux définis pour R;

– un reste acyloxy de formule générale:

$(R_2\,OOC)_p–R_3–COO–$

dans laquelle:

. $R_2$ représente un radical alkyle inférieur ou un reste $(R)_3Sn$ – R ayant la signification donnée ci-avant;

. p est 1 à 2

. $R_3$ est un reste hydrocarboné aliphatique saturé, cycloaliphatique saturé ou aromatique di- ou trivalents.

– un reste sulfonyloxy $R_1SO_3$ – où $R_1$ a la signification donnée ci-avant;

– un reste de formule:

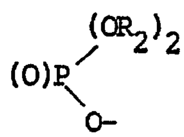

dans laquelle $R_2$ a la signification donnée ci-avant, les deux radicaux $R_2$ pouvant être identiques ou différents;

– un reste de formule:

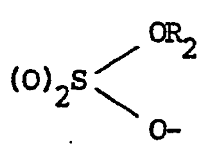

dans laquelle $R_2$ a la signification donnée ci-avant.

3. Procédé selon l'une quelconque des revendications 1 ou 2, caractérisé en ce que la quantité de composé organostannique représente de $1 \times 10^{-4}$ à 5% en poids du polyisocyanate présent dans la solution.

4. Solutions de polyisocyanates dans les solvants organiques inertes stabilisées vis-à-vis de la formation de précipités, caractérisées en ce qu'elles contiennent une quantité efficace d'au moins un dérivé organostannique soluble dans le solvant organique, tel que défini dans les revendications 1 et 2.

## Claims

1. Process for the stabilization of solutions of polyisocyanates in inert organic solvents, against the formation of precipitates, characterized in that an effective quantity is added thereto of at least one organostannic derivative which is soluble in the organic solvent and which corresponds to the general formula:

$(R)_3 – Sn–X$ (I)

in which

– R denotes a hydrocarbon radical containing from 1 to 30 carbon atoms, optionally substituted by a halogen atom or a functional group which is inert towards isocyanate groups, such as alkoxy, carbonyloxyalkyl, nitro or acyl, it being possible for the various radicals to be identical or different;

– X denotes a halogen atom; an acyloxy residue; a sulphonyloxy residue; a residue of oxygen-containing inorganic acid; a residue of formula –Z–Sn $(R)_3$ in which Z denotes an oxygen or sulphur atom and R has the meaning given above; a residue of formula R′–Z in which R′ denotes an alkyl radical containing 1 to 30 carbon atoms, cycloalkyl radicals containing from 5 to 12 carbon atoms, arylalkyl radicals containing 1 to 10 carbon atoms in the alkyl residue, which are optionally substituted by one or more halogen atoms and/or functional groups which are inert towards isocyanate groups, such as alkoxy, carbonyloxyalkyl, nitro or acyl.

2. Process according to Claim 1, characterized in that use is made of organostannic compounds of formula

(I), in which:

– R denotes an alkyl radical containing from 1 to 30 carbon atoms; cycloalkyl radicals containing from 5 to 12 carbon atoms; aryl radicals; arylalkyl radicals containing from 1 to 10 carbon atoms in the alkyl residue, which are optionally substituted by one or more halogen atoms and/or functional groups which are inert towards isocyanate groups such as alkoxy, carbonyloxyalkyl, nitro or acyl;

– X denotes:

– a fluorine, chlorine, or bromine atom;

– an acyloxy residue of formula $R_1$–COO in which $R_1$ denotes an alkyl, cycloalkyl, aryl, or arylalkyl radical, such as those specified for R;

– an acyloxy residue of general formula:

$(R_2OOC)_p–R_3–COO–$

in which:

. $R_2$ denotes a lower alkyl radical or a residue $(R)_3Sn-$ R having the meaning given above;
. p is 1 to 2,
. $R_3$ is a saturated aliphatic, saturated cycloaliphatic or di- or trivalent aromatic hydrocarbon residue;
– a sulphonyloxy residue $R_1SO_3$ – where $R_1$ has the meaning given above;
– a residue of formula:

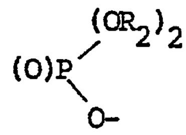

in which $R_2$ has the meaning given above, it being possible for the two radicals $R_2$ to be identical or different;
– a residue of formula:

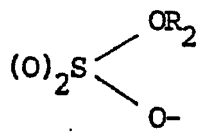

in which $R_2$ has the meaning given above.

3. Process according to either of Claims 1 and 2, characterized in that the quantity of organostannic compound represents from $1 \times 10^{-4}$ to 5% by weight of the polyisocyanate present in the solution.

4. Solutions of polyisocyanates in inert organic solvents, which are stabilized against the formation of precipitates and characterized in that they contain an effective quantity of at least one organostannic derivative which is soluble in the organic solvent, such as specified in Claims 1 and 2.

## Patentansprüche

1. Verfahren zur Stabilisierung von Lösungen von Polyisocyanaten in inerten organischen Lösungsmitteln gegenüber der Bildung von Niederschlägen, dadurch gekennzeichnet, daß man ihnen eine wirksame Menge wenigstens eines in dem organischen Lösungsmittel löslichen Organozinnderivats zusetzt, das der allgemeinen Formel

$(R)_3 - Sn{-}X$ (I)

entspricht, worin:
– R einen Kohlenwasserstoffrest mit 1 bis 30 Kohlenstoffatomen bedeutet, der gegebenenfalls durch ein Halogenatom oder durch eine funktionelle Gruppe substituiert ist, die gegenüber den Isocyanatgruppen inert ist, Alkoxy, Carbonyloxyalkyl, Nitro oder Acyl, wobei die verschiedenen Reste identisch oder verschieden sein können;
– X bedeutet ein Halogenatom, einen Acyloxyrest, einen Sulfonyloxyrest, einen Rest einer Sauerstoffmineralsäure, einen Rest der Formel $-Z{-}Sn\ (R)_3$, worin Z ein Sauerstoff- oder Schwefelatom bedeutet und R die oben angegebene Bedeutung hat; einen Rest der Formel $R'{-}Z$, worin R' einen Alkylrest mit 1 bis 30 Kohlenstoffatomen, Cycloalkylreste mit 5 bis 12 Kohlenstoffatomen, Arylalkylreste mit 1 bis 10 Kohlenstoffatomen im Alkylrest bedeutet, die gegebenenfalls durch ein oder mehrere Halogenatome und/oder funktionelle, gegenüber den Isocyanatgruppen inerte Gruppen substituiert sind, wie Alkoxy, Carbonyloxyalkyl, Nitro oder Acyl.

2. Verfahren gemäß Anspruch 1, dadurch gekennzeichnet, daß man Organozinnverbindungen der Formel (I) verwendet, worin:
– R einen Alkylrest mit 1 bis 30 Kohlenstoffatomen bedeutet; Cycloalkylreste mit 5 bis 12 Kohlenstoffatomen; Arylreste; Arylalkylreste mit 1 bis 10 Kohlenstoffatomen im Alkylrest,
die gegebenenfalls durch ein oder mehrere Halogenatome und/oder gegenüber den Isocyanatgruppen inerte funktionelle Gruppen substituiert sind, wie Alkoxy, Carbonyloxyalkyl, Nitro oder Acyl;
– X bedeutet:
– ein Fluor-, Chlor- oder Bromatom;
– einen Acyloxyrest der Formel $R_1{-}COO$, worin $R_1$ einen Alkyl-, Cycloalkyl-, Aryl-, Arylalkylrest wie diejenigen die für R definiert sind, bedeutet;
– einen Acyloxyrest der allgemeinen Formel: $(R_2OOC)_p{-}R_3{-}COO{-}$
worin:
. $R_2$ einen niedrigen Alkylrest oder einen Rest $(R)_3Sn-$ bedeutet, wobei R die vorstehend angegebene Bedeutung hat;
. p ist 1 bis 2
. $R_3$ ist ein gesättigter aliphatischer Kohlenwasserstoffrest, gesättigter cycloaliphatischer oder 2- oder 3-wertiger aromatischer Rest;
– einen Sulfonyloxyrest, $R_1SO_3-$, worin $R_1$ die vorstehend angegebene Bedeutung hat;
– einen Rest der Formel:

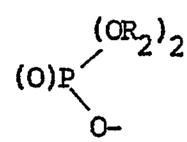

worin $R_2$ die vorstehend angegebene Bedeutung hat, wobei die zwei Reste $R_2$ identisch oder voneinander verschieden sein können;
– einen Rest der Formel:

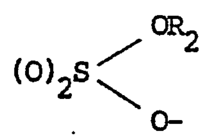

worin $R_2$ die vorstehend angegebene Bedeutung hat.

3. Verfahren gemäß einem der Ansprüche 1 oder 2, dadurch gekennzeichnet, daß die Menge der Organozinnverbindung von $1 \times 10^{-4}$ bis 5 Gew.-%

des in der Lösung vorhandenen Polyisocyanats beträgt.

4. Lösungen von Polyisocyanaten in inerten organischen Lösungsmitteln, stabilisiert gegenüber der Bildung von Niederschlägen, dadurch gekennzeichnet, daß sie eine wirksame Menge wenigstens eines in dem organischen Lösungsmittel löslichen Zinnderivats enthalten, wie es in den Ansprüchen 1 und 2 definiert ist.